# EUROPEAN PATENT SPECIFICATION

(11) **EP 2 628 461 B1**
(45) Date of publication and mention of the grant of the patent: **30.07.2014**
(21) Application number: 13151237.8
(22) Date of filing: 15.01.2013
(51) Int. Cl.: A61C 1/08, A61B 19/00

(54) **Dental handpiece with a gyroscopic position sensor**
Zahnmedizinisches Handstück mit einem gyroskopischen Positionssensor
Pièce à main dentaire avec capteur de position gyroscopique

(30) Priority: 15.02.2012 IT MI20120209
(43) Date of publication of application: 21.08.2013
(73) Proprietor: I.D.I. Evolution S.r.l., 20900 Monza (IT)
(72) Inventor: Piantoni, Angiolino, 20855 Lesmo (IT)
(74) Representative: Martegani, Franco

(56) References cited:
- EP-A2- 0 312 171
- WO-A1-2011/089606
- GB-A- 2 181 845

## Description

The present invention relates to a dental instrument with a gyroscopic position sensor.

Numerous instruments are used by operators in the dental field. Specific cutting units are used in the positioning, for example, of so-called "dental implants", i.e. an artificial screw-shaped root in titanium or similar metal, which is inserted in the alveolar bone to substitute the roots of missing teeth.

These dental implants are in fact provided with a threaded internal cavity for positioning dental superstructures, wherein the term "dental superstructure" (SD) relates to the prosthetic components to be screwed into the internal thread of the implant and whose characteristics allow the fixing of the screwed or cemented prosthetic rehabilitation.

It should be noted that the term "prosthetic rehabilitation" (RP) indicates hereunder the dental prosthesis that substitutes a single tooth, various teeth or a whole arch held and fixed to dental implants previously inserted in the alveolar bone through outpatient surgery.

For the implementation of a prosthetic rehabilitation, for example, there is a first phase which consists in positioning dental implants in the alveolar bone with an orientation that depends on the availability of the bone.

The implementation phase envisages effecting cuttings or holes in the alveolar bone of the patient.

It should be noted that only rarely is it possible to insert dental implants parallel to each other and consequently resort must be made to specific dental superstructures whose characteristics allow the lack of parallelism of dental implants to be compensated.

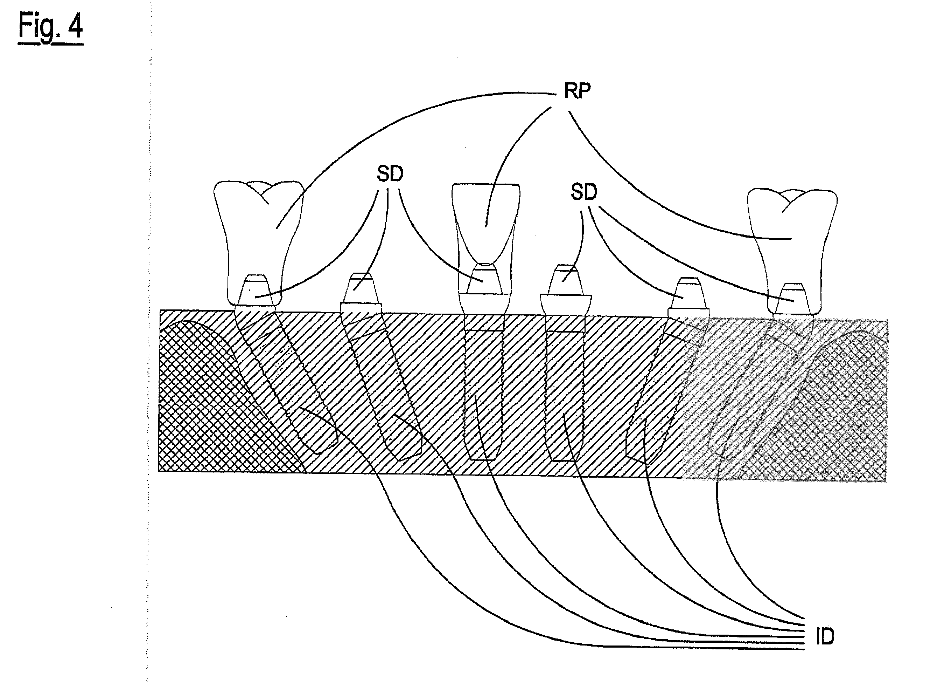

This problem is synthetically illustrated in the enclosed Figure 4.

The prosthetic rehabilitation fixed to the superstructures by screwing (screwed prosthesis) or stabilized by means of dental cement (cemented prosthesis), is then formed on these devices, which can be produced in series or individually.

The anatomical availability, capacity of the operator or clinical indications create a variability in the result which is not compatible with a production in series of parallelizing superstructures which conform to all solutions.

It should be noted that implants may come to be positioned with a reciprocal angle of 90° and consequently at least 45 different types of superstructures should be produced for an exact compensation (one for each single inclination angle).

Companies present in the field (mainly for technical reasons of cost reduction) all agree in supplying a limited range of angulations (e.g.: 10°-20°-30°...) which makes it necessary to deviate from the ideal condition of parallelism, as compromise solutions must be accepted.

The same problem arises in the use of other dental instruments in which an action must be exerted according to a predetermined orientation deriving from problems associated with the orientation according to which the intervention on the patient must be effected.

WO-A-2011 089606 describes a dental hand-piece with a gyroscopic position sensor mounted thereon.

The main objective of the present invention therefore relates to the development of a device which, when applied to a dental instrument, allows this drawback to be minimized.

This objective is achieved with a dental instrument having a gyroscopic position sensor according to independent claim 1.
In short, the idea at the basis of the invention is to use a gyroscopic position sensor associated with the handpiece of the dental instrument which is able to provide the operator with information relating to the spatial position of the tool regardless of the orientation of the instrument during use. A support is constrained to such dental handpiece, on which a position gyroscopic sensor is positioned. According to the invention an angular adapter of the removable type is interposed between the gyroscopic sensor and the support, which angular adapter determines the spatial disposition of an axis of the gyroscopic sensor to be parallel to an operative axis of the tool.

In this way, the operator is able to carry out operations according to the capacity of the instrument regardless of his subjective ability or experience.

The operator is in fact able to monitor the operating direction of the tool at each moment and operate according to the direction previously selected in relation to the specific requirements.

The structural and functional characteristics of the invention, as also its advantages with respect to the known art, can be clearly understood from the following description, referring to the enclosed drawings, which show a possible practical embodiment of the invention itself.

In the drawings:
- figure 1 illustrates a scheme of a cutting handpiece equipped with a sensor according to the present invention, partially exploded, in a complete operational group;
- figure 2 illustrates a detail of part of the dental instrument equipped with the sensor of figure 1;
- figure 3 illustrates a variant of what is shown in figure 1;
- figure 4 shows a combination of a dental implant, superstructure and prosthesis for which the instrument of the present invention is used;
- figures 5, 6 and 7 show details of the support and adaptor which form part of the instrument of the invention illustrated in the previous figures;
- figure 8 shows a block scheme of the hardware of the invention illustrating the connections between the parts in play and dependency between them;
- figure 9 shows a block scheme of the calculation algorithm of the angles;
- figures 10 and 11 shows graphs indicating the extent in degrees of the angle between the direction of the cutter at the moment in which it effects "zero" and the direction of the "running" cutter.

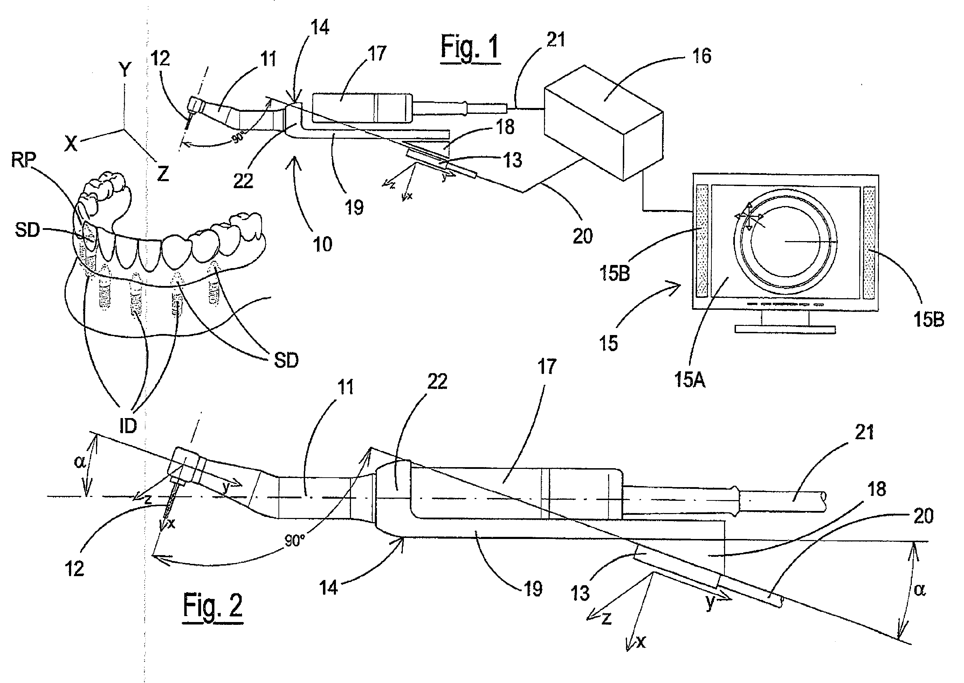

With reference to figures 1 and 2, these show an embodiment of a dental instrument with an application device of a sensor according to the present invention, indicated as a whole with 10, situated within a complete operating group.

The dental instrument 10 comprises a dental handpiece 11 equipped with a drilling tool 12 which in this example is a cutter tip, but can also be another tool.

The instrument 10 also comprises a position sensor 13 which is associated with a handpiece 11 by means of a specific support 14 according to the invention.

The sensor consists of a gyroscopic position sensor 13 and serves to determine and regulate the spatial direction of the drilling tool 12 with respect to the bone tissue on which the hole or operation required is to be effected. The sensor used is a completely digital sensor.

For this purpose, further aid is provided by a processor 16 of the signal transmitted by the position sensor 13 and representation means 15 of the spatial orientation of the drilling tool 12.

The term "spatial orientation" in this description indicates the spatial orientation of the tool with respect to a tern of Cartesian axes. The processor 16 of the signal transmitted by the position sensor 13 exerts the function of processing the signals generated by the position sensor 13, for example the inclination angles, to allow them to be represented graphically or compared with thresholds of known values.

The sensor 13 and processor 16 are connected by means of a communication line, which can be a wireline 20 or wireless (radio signals or the like), or another form of communication line.

Furthermore, the processor 16 also effects these comparisons and generates alarm signals and/or intervenes on a micromotor 17 which is associated with the handpiece 11 to complete the dental instrument 10, connected by means of a respective wire 21.

The processor 16 can be a separate component or it can be integrated in a unit that exerts various functions, also not only pertinent to the control of the instrument 10. The processor 16 for example can be integrated in an electronic processor, such as a personal computer or the like.

The representation means 15, in the present example, comprise both visualization means 15A and acoustic signalling means 15B.

The visualization means 15A in turn comprise a screen, such as, for example, that of an electronic processor or the like; analogously, the acoustic signalling means 15B comprise a speaker, either separate or part of an electronic processor.

The association between the position sensor 13 and handpiece 11 is effected, as already mentioned, by means of a specific support 14 which carries, according to the invention, an angular adapter 18, for example in the form of a wedge.

The adapter 18 is positioned on the support 14 either removably or integrally.

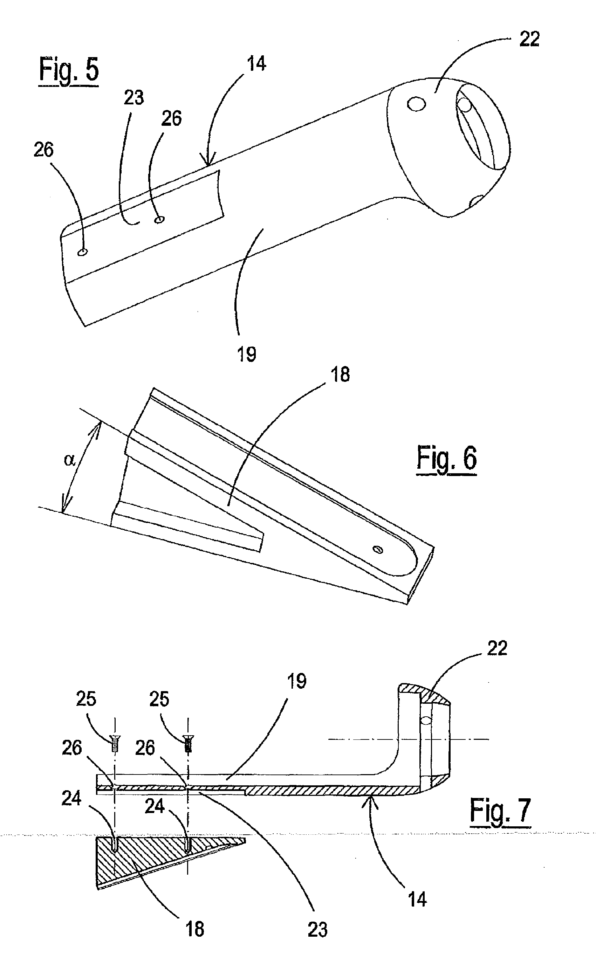

Figures 5, 6 and 7 show details of the support and adapter forming part of the instrument of the invention illustrated in the previous figures.

In particular, figure 5 shows a perspective view of the support 14 which, in the example shown, is in the form of an elongated slab-shaped extension 19 which extends from a perforated circular crown 22, forming a shaped hooking head, for fixing with respect to an end of the dental handpiece 11. The micromotor 17 is also connected to this end.

The slab or elongated slab-shaped extension 19 in turn contains, above and facing outwardly, a hollow seat 23 which receives the adaptor 18 on which the gyroscopic sensor 13 is positioned, which also has an appropriate insertion-shaping, complementary to that on the adaptor. The adaptor 18, in this non-limiting example, has two holes 24 suitable for receiving fixing screws 25 which, before being engaged, pass through additional holes 26 situated in the hollow seat 23 of the elongated slab 19.

This removable arrangement of the adaptor 18 makes it possible to vary the type of adaptor 18 in relation to the angle α necessary for the correct positioning.

The adaptor 18 is selected so as to align the axis x of the gyroscopic sensor 13 with the axis X of the cutter 12 assembled on the handpiece 11.

In this way, the measurement of the angle is independent of the rotations around the axis X of the cutter 12.

The positioning of the adaptor 18 with respect to the support 14 can be effected in various other ways: there can, for example, be suitable hooks on one or both, or, more simply, they can be associated with the use of a simple insertion-coupling in a suitable seat, as previously illustrated, etc. In any case, engagement means must be provided, that can be reciprocally and freely released, which allow the adaptor 18 to be substituted with an angle α selected. This angle α is selected in relation to the type of handpiece envisaged by each producer with a different angulation of the positioning head of the tool.

It should be noted that the relative position of the position sensor 13 with respect to the handpiece 11 advantageously does not influence the detection of the spatial orientation of the drilling tool 12 when operating, for the reasons that will be discussed hereunder and, in short, relating to the presence of a preliminary reciprocal positioning phase with a prefixed angle.

This preliminary positioning phase with a prefixed angle is effected each time the sensor 13 is associated again with the handpiece 11, or on the support 14 and on the relative preselected adaptor 18.

In this way, the position of the sensor 13 is suitably associated with the rotation axis of the tool, also assuming the substitution of the type of handpiece or modification of the position of the sensor thereon.

Following the initial calibrated positioning phase with a prefixed angle, the operator can use the dental instrument 10, each time selecting a system of Cartesian reference axes (which for the sake of simplicity is defined hereunder with the term "zero value") depending on the specific operating requirements, transmitting these values to the processor 16.

With brief reference to the visualization means 15A, these show the spatial orientation of the tool used 12 with respect to a Cartesian reference tern, coinciding with that established and called "zero value".

This visualization shows, in particular, the inclination angles of the tool used, for example the drilling tool 12, with respect to the three axes of said Cartesian reference tern, so that the operator, when holding the handpiece 11, can accurately follow on the visualization means 15A, the progressive inclination of the tool 12 after moving the handpiece 11.

The Cartesian reference tern (with respect to which the inclination angles are measured and shown) is established, advantageously each time, with the setting phase of "zero value", which allows the operator to have a reference system congruent with the operating reality (for example the spatial orientation of the implant, patient, etc.).

In practice, the operator holds the handpiece 11 and rests the tip of the tool 12 on the part to be operated; holding the handpiece 11 still for a few seconds, he then effects the setting phase of "zero value" of the instrument 10: in this phase, the processor 16 reveals and memorizes the spatial orientation (for example the inclinations with respect to the Cartesian axes of the instrument or the force of gravity components on the three Cartesian axes of the instrument) revealed by the sensor 13 and memorizes them to subsequently use them as "zero value", i.e. the reference with respect to which the further spatial directions revealed by the sensor 13 will be measured.

In this way, for example, the operator can work easily on a patient who has his jaw open inclined at, for example, 15° with respect to the horizontal axis.

If a hole with an inclination of 17° is to be effected in the bone of said jaw, in fact, the operator does not have to worry about taking into consideration the angle at which the patient's jaw is inclined, but can simply rest the tip of the tool 12 in the point in which the hole is to be initiated, effecting the "zero value" setting phase of the instrument 10 on the processor 16, holding the handpiece 11 in the desired position, then moving the handpiece until it reaches the desired inclination (specifically 17° in this example) measured with respect to the "zero value" set (which therefore consists in an actual Cartesian reference axis system), following the progressive inclination of the tip of the tool 12, on the visualization means 15A.

When the tip of the tool 12 is spatially positioned correctly, the operator activates the motor 17 which moves the tool 12 and begins the drilling or cutting or other operation.

An improvement envisages that during (the whole or part of) the operating phase of the tool 12 of the handpiece 11, its position is substantially continuously monitored. This is achieved by ensuring that the position sensor 13 continuously transmits signals to the processor 16 which converts them and adapts them to be continuously displayed on the visualization means 15A, so that the operator can keep the spatial orientation of the tool 12 under control, also while operating, in order to verify whether it is positioned correctly.

Said visualization when operating also takes place with reference to the tern of Cartesian axes determined during the setting phase of the "zero value" indicated above.

A further improvement lies in the fact that tolerance thresholds are pre-established and/or can be set and, if these values are exceeded, a sonorous and/or visible alarm is activated: assuming that the operator must effect a hole inclined by 17° with respect to one (or more) axes of the Cartesian reference system, the operator can set a tolerance threshold, for example of ± 2°, so that outside this tolerance range (from 15° to 19° in the example cited), a sonorous signal is emitted (by acoustic means 15B) and/or visibly (through the visualization means 15A).

An advantageous variant consists in the fact that the sonorous signal is emitted continuously, even within the tolerance values: in this case, the signal advantageously has a variable intensity and/or frequency (for example, increasing) proportional to the distance from the centre of the tolerance range (therefore from 17° in this example) so that the operator can follow the operation with his eyes without losing control of the inclination, which he can follow, indirectly, through the sonorous signal thus generated. Another solution envisages that, if the first tolerance range is exceeded by a certain amount, there is an intervention on the motor 17 that activates the tool 12, to automatically switch it off.

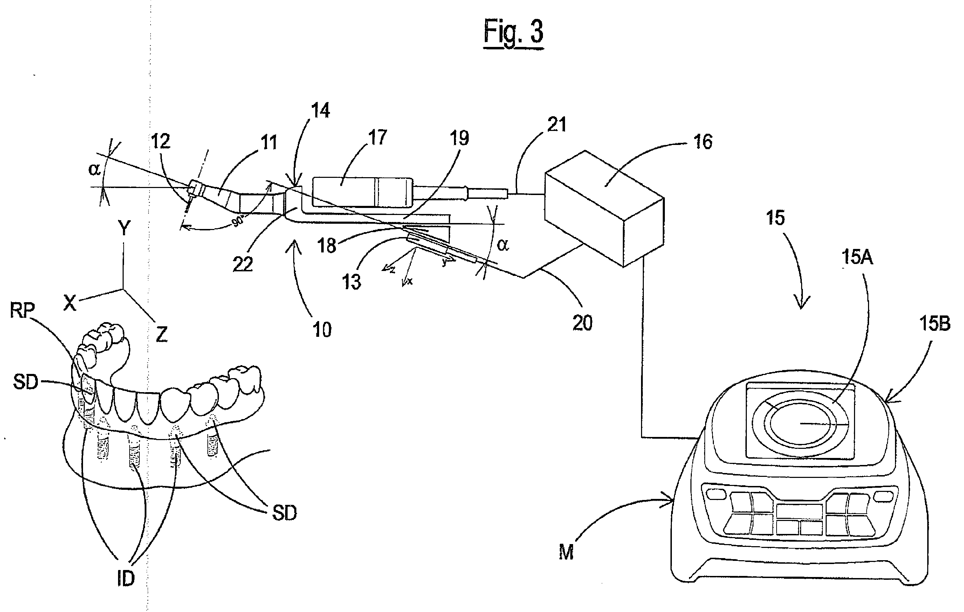

A variant of the instrument 10 is shown in figure 3.

In this variant, the same numbers indicate the same parts, and consequently no further reference is made to these for the sake of conciseness.

In this variant, the handpiece 11 is activated by the motor M according to the disclosures provided in patent EP 1842484 by the same owner.

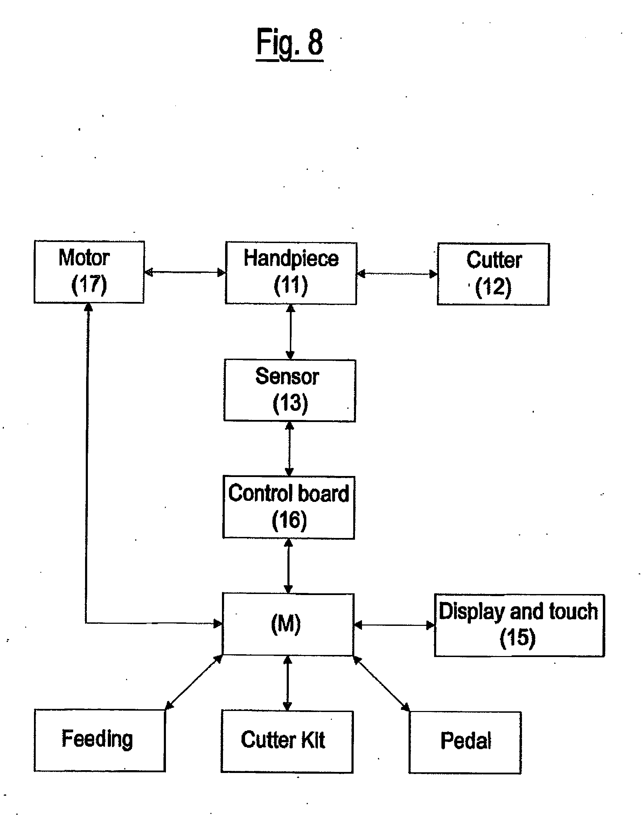

Figure 8 shows a block scheme of the hardware of the invention, which illustrates the connections between the parts in play and dependency between them.

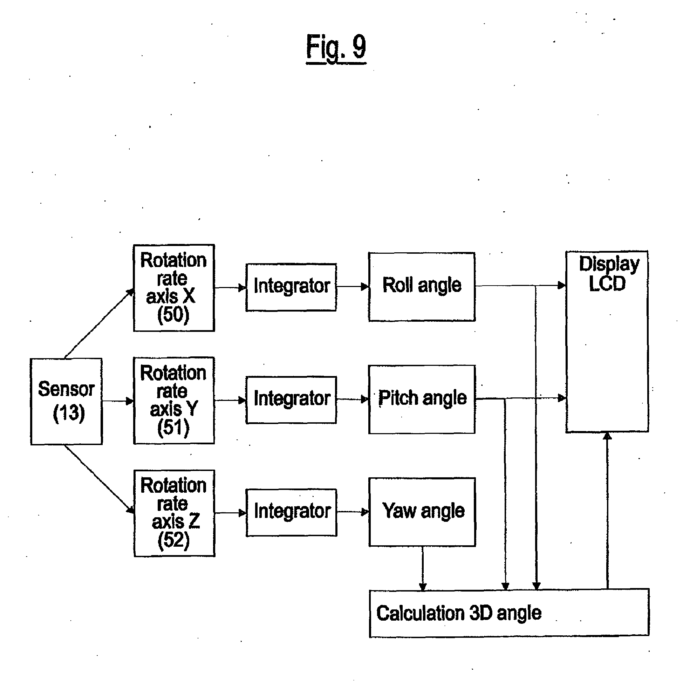

Figure 9 shows the block scheme of the calculation algorithm of the angles. The sensor 13 is, as already indicated, a three-axis electronic gyroscopic sensor. It is equipped with a fast serial interface and is capable of supplying the angular rotation rates of the sensor itself around its three axes x, y, z (indicated in the three blocks 50, 51, 52, called "rotation rate").

The Control Board 16 acquires the data relating to the gyroscopic rates supplied by the sensor 13, through a fast serial 53.

The Control Board 16 effects the calculation of the angles and prepares the data for the system M (the calculation of the angles is effected through the integral calculation with time of the angular velocity trends).

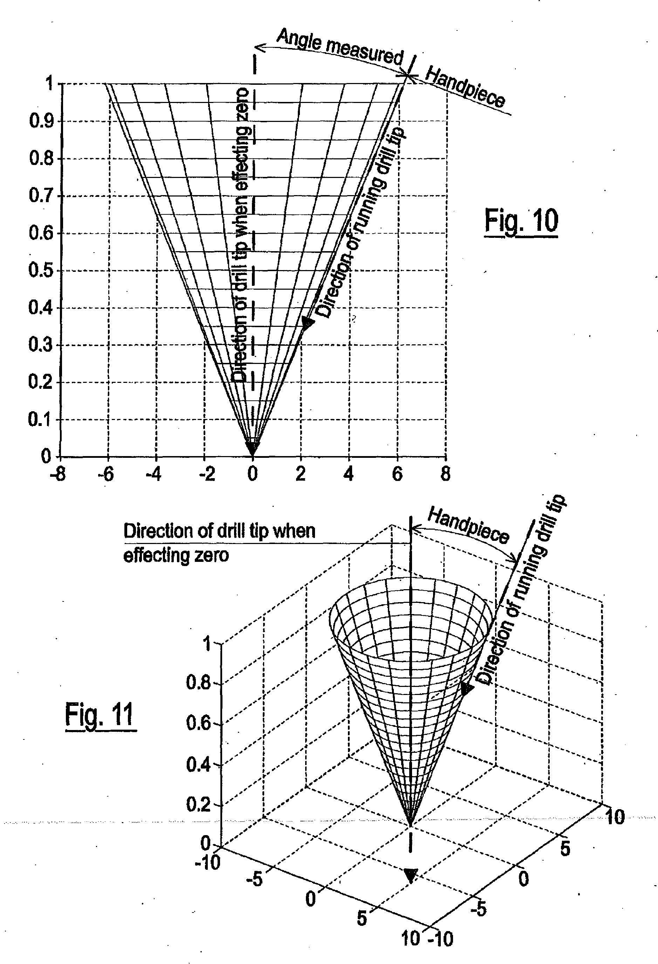

The Pitch, Roll and Yaw angles are then recombined mathematically to reveal the angle 3D, i.e. the measurement in degrees of the angle between the direction of the cutter at the moment in which "zero" is effected and the "running" direction of the cutter, in the plane of the two lines indicating the initial and running direction (see figures 10 and 11).

Numerous variants are possible, that should all be considered as being part of the present invention.

Instead of the cutter tip, for example, there could equivalently be a drill tip or similar drilling tool that can be used for effecting other types of holes in the dental field.

Furthermore, in the place of a handpiece 11, another analogous dental instrument can be adopted, such as a drill or other tool for dental use.

Yet again, instead of a range of values, there could be two, three or more with one or more alarms that can be selectively activated depending on the extent to which said threshold values are exceeded.

With respect to the latter, it may be convenient to have only pre-established values, or only values that can be set by the operator, or a combination of both, for example, to be able to regulate the instrument 10 more rapidly if routine operations are to be effected and with greater precision when operations different from normal procedures, are to be effected.

The scope of the invention is defined by the following claims.

## Claims

1. A dental instrument (10) with application device of a position gyroscopic sensor, wherein the dental instrument is a dental handpiece (11), equipped with a tool-bearing head and relevant tool (12) to which a micro-motor (17) is associated, wherein a support (14) is constrained to said dental handpiece (11), on which said position gyroscopic sensor (13) is positioned, **characterized in that** an angular adapter (18) of the removable type is interposed between said gyroscopic sensor (13) and said support (14), which angular adapter (18) determines the spatial disposition of an axis (x) of said gyroscopic sensor (13) to be parallel to an operative axis (X) of said tool (12).

2. The dental instrument according to claim 1, **characterized in that** said support (14) also includes a shaped hooking head (22) to said dental handpiece (11) and a slab-shaped extension (19) as a constraint of said sensor (13) and adapter (18).

3. The dental instrument according to claim 2, **characterized in that** said support (14) also includes a shaped hooking head with a ring-shaped end (22) which can be inserted on a complementary end of said dental handpiece (11) before being hooked to said micro-motor (17).

4. The dental instrument according to one or more of the previous claims, **characterized in that** said support (14) includes a housing seat (23) and stable positioning of said angular adapter of the removable type (18).

5. The dental instrument according to claim 4, **characterized in that** said seat (23) and said adapter (18) include reciprocal engagement means, which can be freely unfastened (25,24,26).

6. The dental instrument according to one or more of the previous claims, **characterized in that** said adapter (18) is shaped in the form of a wedge.

7. The dental instrument according to one or more of the previous claims, **characterized in that** said adapter (18) consists of an extension of said support.

8. The dental instrument according to one or more of the previous claims, **characterized in that** it also includes means (15) for the representation of the spatial disposition of said tool.

9. The dental instrument according to claim 8, **characterized in that** said representation means of the spatial disposition of said tool comprise visualization means (15A) and/or acoustic signalling means (15B).

10. The dental instrument according to claim 8 or 9, **characterized in that** said dental instrument also includes a processor (16) of a signal transmitted by said position gyroscopic sensor (13).

## Patentansprüche

1. Zahnmedizinisches Instrument (10) mit einer Anwendungsvorrichtung eines gyroskopischen Positionssensors, wobei das zahnmedizinische Instrument ein zahnmedizinisches Handstück (11) ist, das mit einem ein Werkzeug tragenden Kopf und einem relevanten Werkzeug (12) ausgestattet ist, dem ein Mikromotor (17) zugeordnet ist, wobei ein Träger (14) an dem zahnmedizinischen Handstück (11) eingezwängt ist, an dem der gyroskopische Positionssensor (13) positioniert ist, **dadurch gekennzeichnet, dass** ein Winkeladapter (18) des entfernbaren Typs zwischen dem gyroskopischen Sensor (13) und dem Träger (14) angeordnet ist, wobei der Winkeladapter (18) die räumliche Anordnung einer Achse (x) des gyroskopischen Sensors (13) so bestimmt, dass sie parallel zu einer operativen Achse (X) des Werkzeugs (12) ist.

2. Zahnmedizinisches Instrument nach Anspruch 1, **dadurch gekennzeichnet, dass** der Träger (14) auch einen geformten Hakenkopf (22) an dem zahnmedizinischen Handstück (11) und eine plattenförmige Verlängerung (19) als eine Einzwängung des Sensors (13) und Adapters (18) aufweist.

3. Zahnmedizinisches Instrument nach Anspruch 2, **dadurch gekennzeichnet, dass** der Träger (14) auch einen geformten Hakenkopf mit einem ringförmigen Ende (22) aufweist, das an einem komplementären Ende des zahnmedizinischen Handstücks (11) eingesetzt werden kann, bevor es an den Mikromotor (17) eingehakt wird.

4. Zahnmedizinisches Instrument nach einem der mehreren der vorhergehenden Ansprüche, **dadurch gekennzeichnet, dass** der Träger (14) einen Gehäusesitz (23) und eine stabile Positionierung des Winkeladapters des entfernbaren Typs (18) aufweist.

5. Zahnmedizinisches Instrument nach Anspruch 4, **dadurch gekennzeichnet, dass** der Sitz (23) und der Adapter (18) ein hin- und herbewegliches Eingriffsmittel aufweisen, das frei unbefestigt (25, 24, 26) sein kann.

6. Zahnmedizinisches Instrument nach einem oder mehreren der vorhergehenden Ansprüche, **dadurch gekennzeichnet, dass** der Adapter (18) in der Form eines Keiles geformt ist.

7. Zahnmedizinisches Instrument nach einem oder mehreren der vorhergehenden Ansprüche, **dadurch gekennzeichnet, dass** der Adapter (18) aus einer Verlängerung des Trägers besteht.

8. Zahnmedizinisches Instrument nach einem oder mehreren der vorhergehenden Ansprüche, **dadurch gekennzeichnet, dass** es auch ein Mittel (15) für die Repräsentation der räumlichen Anordnung des Werkzeugs aufweist.

9. Zahnmedizinisches Instrument nach Anspruch 8, **dadurch gekennzeichnet, dass** das Repräsentationsmittel der räumlichen Anordnung des Werkzeugs ein Visualisierungsmittel (15a) und/oder ein Akustiksignalmittel (15b) umfasst.

10. Zahnmedizinisches Instrument nach einem der Ansprüche 8 oder 9, **dadurch gekennzeichnet, dass** das zahnmedizinische Instrument auch einen Prozessor (16) eines Signals umfasst, das von dem gyroskopischen Positionssensor (13) übertragen wird.

## Revendications

1. Instrument dentaire (10) avec dispositif d'application d'un capteur de position gyroscopique, dans lequel l'instrument dentaire est une pièce à main dentaire (11), équipée d'une tête porte-outil et d'un outil approprié (12) auquel un micromoteur (17) est associé, dans lequel un support (14) est fixé sur ladite pièce à main dentaire (11), sur lequel est positionné ledit capteur de position gyroscopique (13), **caractérisé en ce qu'**un adaptateur angulaire (18) de type amovible est intercalé entre ledit capteur gyroscopique (13) et ledit support (14), lequel adaptateur angulaire (18) détermine la disposition spatiale d'un axe (x) dudit capteur gyroscopique (13) pour qu'elle soit parallèle à un axe fonctionnel (x) dudit outil (12).

2. Instrument dentaire selon la revendication 1, **caractérisé en ce que** ledit support (14) comprend aussi une tête d'accrochage façonnée (22) sur ladite pièce à main dentaire (11) et une extension en forme de plaque (19) en tant que contrainte dudit capteur (13) et dudit adaptateur (18).

3. Instrument dentaire selon la revendication 2, **caractérisé en ce que** ledit support (14) comprend aussi une tête d'accrochage façonnée ayant une extrémité de forme annulaire (22) qui peut être insérée sur une extrémité complémentaire de ladite pièce à main dentaire (11) avant d'être accrochée audit micromoteur (17).

4. Instrument dentaire selon l'une ou plusieurs des revendications précédentes, **caractérisé en ce que** ledit support (14) comprend un siège de logement (23) et un positionnement stable dudit adaptateur angulaire du type amovible (18).

5. Instrument dentaire selon la revendication 4, **caractérisé en ce que** ledit siège (23) et ledit adaptateur (18) comprennent un moyen de mise en prise réciproque, qui peut être détaché librement (25, 24, 26).

6. Instrument dentaire selon l'une ou plusieurs des revendications précédentes, **caractérisé en ce que** ledit adaptateur (18) a la forme d'un coin.

7. Instrument dentaire selon l'une ou plusieurs des revendications précédentes, **caractérisé en ce que** ledit adaptateur (18) consiste en une extension dudit support.

8. Instrument dentaire selon l'une ou plusieurs des revendications précédentes, **caractérisé en ce qu'**il comprend en outre un moyen (15) pour la représentation de la disposition spatiale dudit outil.

9. Instrument dentaire selon la revendication 8, **caractérisé en ce que** ledit moyen de représentation de la disposition spatiale dudit outil comprend un moyen de visualisation (15A) et/ou un moyen de signalisation acoustique (15B).

10. Instrument dentaire selon la revendication 8 ou 9, **caractérisé en ce que** ledit instrument dentaire comprend aussi un dispositif de traitement (16) d'un signal transmis par ledit capteur de position gyroscopique (13).
